# EUROPÄISCHE PATENTSCHRIFT

(11) **EP 0 461 531 B1**
(45) Veröffentlichungstag und Bekanntmachung des Hinweises auf die Patenterteilung: **14.09.1994**
(21) Anmeldenummer: 91109149.4
(22) Anmeldetag: 05.06.1991
(51) Int. Cl.: C07C 205/44, C07C 201/16

(54) **Verfahren zur Reinigung von 2-Chlor-5-nitrobenzaldehyd oder dessen Acetalen**
Process for the purification of 2-chloro-5-nitrobenzaldehyde or their acetals
Procédé de purification du 2-chloro-5-nitrobenzaldéhyde ou de leurs acétals

(30) Priorität: 15.06.1990 DE 4019049
(43) Veröffentlichungstag der Anmeldung: 18.12.1991
(73) Patentinhaber: BASF Aktiengesellschaft, 67063 Ludwigshafen (DE)
(72) Erfinder: Schaefer, Bernd, Dr., W-6749 Dierbach (DE); Freund, Wolfgang, Dr., W-6730 Neustadt (DE); Reissenweber, Gernot, Dr., W-6737 Boehl-Iggelheim (DE)

(56) Entgegenhaltungen:
- EP-A- 0 118 862
- EP-A- 0 305 812
- EP-A- 0 305 812
- MONATSHEFTE FUR CHEMIE. Bd. 25, 1904, WIEN AT Seiten 365 - 374; PAUL COHN ET ALBERT BLAU: 'über substituierte Benzaldehyde'
- JOURNAL OF THE CHEMICAL SOCIETY. Januar 1926, LONDON GB Seiten 147 - 155; HERBERT HENRY HODGSON ET AL.: 'Chloro-derivatives of m-Hydroxybenzaldehyde.'

## Beschreibung

Die vorliegende Erfindung betrifft ein Verfahren zur Reinigung von 2-Chlor-5-nitrobenzaldehyd oder dessen Acetalen aus ihren Isomerengemischen bei Temperaturen von (-10) bis +140°C durch Suspension.

Aus Monatshefte, Bd. 25, 365-374 (1904) ist bekannt, daß 2-Chlor-5-nitro-benzaldehyd von seiner Herstellung her oft mit einem zweiten Isomeren, 2-Chlor-3-nitrobenzaldehyd verunreinigt ist.

Zur Reinigung des gewünschten 2-Chlor-5-nitrobenzaldehyds kann dieses aus verschiedenen Lösungsmitteln umkristallisiert werden z.B. aus verd. Ethanol, Chloroform/Ligroin nach Liebigs Ann., Bd. 272, 148-140 (1893) oder verd. Essigsäure nach J. Chem. Soc. 147-155 (1926). Dies ist sehr aufwendig und muß unter Umständen ein zweites Mal wiederholt werden (siehe Liebigs Ann., Bd. 272, 147-156 (1893). Darüber hinaus ergeben sich erhebliche Verluste an dem gewünschten Produkt. Ferner sind beträchtliche Mengen an Lösungsmittel nötig. Im technischen Maßstab sind folglich zusätzliche Maßnahmen in Hinblick auf die Rückgewinnung der Lösungsmittel erforderlich.

Aus EP-A-305 812 und EP-A-118 862 sind Reinigungsverfahren von m-Nitrobenzaldehyd bzw. m-Nitrobenzolsäuremethylester durch Behandlung mit Emulgatoren bekannt.

In der DE-A-37 28 826 wird die Reinigung von m-Nitrobenzaldehyd mit Wasser in Gegenwart eines Emulgators durch Erhitzen der Suspension auf Rückfluß beschrieben.

Dieses Verfahren hat die Nachteile, daß man in der Regel auf Rückfluß erhitzen muß, um eine homogene Suspension zu erzielen und daß man nach der Reinigung die unerwünschten Stellungsisomeren sowie die oft toxikologisch nicht unbedenklichen Emulgatoren in der wässrigen Phase erhält.

Darüber hinaus versagt das Verfahren gänzlich beim Versuch der Reinigung von 2-Chlor-5-nitrobenzaldehyd.

Der vorliegenden Erfindung lag daher die Aufgabe zugrunde, den zuvor genannten Nachteilen abzuhelfen.

Demgemäß wurde ein neues und verbessertes Verfahren zur Reinigung von 2-Chlor-5-nitrobenzaldehyd oder deren Acetalen aus ihren Isomerengemischen bei Temperaturen von (-10) bis +140°C gefunden, welches dadurch gekennzeichnet ist, daß man die Isomerengemische in einem Lösungsmittel oder einem Lösungsmittelgemisch suspendiert.

Das erfindungsgemäße Verfahren zeichnet sich dadurch aus, daß die Chlornitrobenzaldehydisomerengemische (2,5- und 2,3-Isomeres) oder deren Acetale in Form einer Suspension gereinigt wird. Unter Suspension (Vgl. Römpp-Lexikon) versteht man ein heterogenes Gemisch, bestehend aus einer flüssigen und einer festen Phase, deren Anteil unterschiedlich groß sein kann.

Erfindungsgemäß beträgt der Feststoffanteil im allgemeinen 50 bis 99 Gew.-%, bevorzugt 60 bis 98 Gew.-% und ganz besonders bevorzugt 70 bis 95 Gew.-%, bezogen auf das Trockengewicht des eingesetzten Chlornitrobenzaldehydisomerengemisches oder deren Acetalen.

Die verwendeten Lösungsmittel lassen sich in 2 Gruppen einteilen: Lösungsmittel der Gruppe A: C₁- bis C₂₀-Alkanole, bevorzugt C₁- bis C₈-Alkanole, besonders bevorzugt C₁- bis C₄-Alkanole wie Methanol, Ethanol, n-Propanol und i-Propanol, Ketone, wie Aceton, Diethylketon und Ethylmethylketon, Ester wie Essigsäureethylester und Essigsäuremethylester, Ether, bevorzugt cyclische Ester wie Tetrahydrofuran und Tetrahydropyran, aromatische Kohlenwasserstoffe wie Benzol, Toluol, o-, m- und p-Xylol oder Mischungen dieser Stoffklassen und Lösungsmittel der Gruppe B: aliphatische Kohlenwasserstoffe, wie Pentane, Hexane, Heptane, Cyclohexan, Benzine, Petrolether, Wasser oder Mischungen dieser Stoffklassen.

Im allgemeinen sind 3 Verfahrensweisen möglich.
1. Der rohe Chlornitrobenzaldehyd wird in einem Lösungsmittel der Gruppe A suspendiert und ein Lösungsmittel der Gruppe B zugefahren.
   Der Anteil der festen Phase in der Suspension aus Chlornitrobenzaldehyd und dem Lösungsmittel der Gruppe A beträgt im allgemeinen 10 Gew. -%, bevorzugt 30 Gew.-% und ganz bevorzugt 60 Gew. -%, bezogen auf das Gewicht der Suspension.
   Der Anteil der festen Phase in der Suspension ist im entscheidenden Maß von dem Anteil an leichter löslichen Verunreinigungen, insbesondere dem Anteil von 2-Chlor-3-nitrobenzaldehyd, abhängig.
2. Der rohe Chlornitrobenzaldehyd wird in einem Lösungsittel der Gruppe B suspendiert und ein Lösungsmittel der Gruppe A zugefahren.
3. Der rohe Chlornitrobenzaldehyd wird mit einem Lösungsmittelgemisch der Gruppe A und B behandelt.

Die Angaben über den Anteil der festen Phase in der Suspension aus Chlornitrobenzaldehyd und den Lösungsmitteln der Gruppe A und B gelten auch für die Verfahrensweise 2 und 3 sinngemäß.

Statt des trockenen Chlornitrobenzaldehyds kann auch feuchtes, also im Bereich von 0 bis 500 Gew.-%, bevorzugt 0 bis 100 Gew.-% Wasser zum Chlornitrobenzaldehyd eingesetzt werden. Gegebenenfalls ist diese Wassermenge bei dem Lösungsmittelgemisch aus der Gruppe A und B zu berücksichtigen. Im gleichen Sinne können auch die bei der Bearbeitungstemperatur festen Acetale, insbesondere das Dimethylacetal und das Glykolacetal, Verwendung finden. Diese Bearbeitungstemperatur liegt im allgemeinen im Bereich von (-10) bis +140°C, bevorzugt von 0 bis 100°C und ganz besonders bevorzugt im Bereich von 10 bis 40°C.

Im allgemeinen arbeitet man im pH-Bereich von 2 bis 12, bevorzugt im pH-Bereich von 4 bis 10, besonders bevorzugt im pH-Bereich von 6 bis 8.

Unter rohem Chlornitrobenzaldehyd versteht man ein Isomerengemisch aus 2-Chlor-5-nitrobenzaldehyd und 2-Chlor-3-nitrobenzaldehyd, wobei es sich bei der Verunreinigung im wesentlichen um 2-Chlor-3-nitrobenzaldehyd handelt, mit einem Anteil von 0,1 bis 15 Gew.%, bevorzugt im Bereich von 0,1 bis 12 Gew.% und ganz besonders bevorzugt im Bereich von 0,1 bis 8 Gew.% bezogen auf das Trockengewicht des Isomerengemischs. Unter den entsprechenden Acetalen sind insbesondere das Dimethylacetal und Glykolacetal bevorzugt.

Das erfindungsgemäße Verfahren kann drucklos oder unter Druck bei 1 bis 50 bar, bevorzugt bei 1 bis 10 bar kontinuierlich oder diskontinuierlich durchgeführt werden. Die in rohen Chlornitrobenzaldehydisomerengemischen oder den Acetalisomerengemischen enthaltenen Verunreinigungen werden zu 50 Gew. -%, bevorzugt zu 80 Gew.-% abgereichert.

Gelegentlich muß ein Kompromiß zwischen Ausbeute und Isomerenreinheit geschlossen werden. Im Allgemeinen liegen die Ausbeuten zwischen 80 und 95 % und die Isomerenreinheit über 98 %.

### Beispiele

### Beispiel 1

10 g des Isomerengemisches aus 2-Chlor-5-nitrobenzaldehyds und 2-Chlor-3-nitrobenzaldehyd (GC: 91 Fl.% 2,5 Isomeres, 8,7 Fl.% 2,3 Isomeres) werden mit 100 ml eines Methanol/Wasser-Gemischs (Volumenverhältnis: 1 : 1) suspendiert, 30 min gerührt und abgesaugt. Man erhält 8,5 g (93 %) 2-Chlor-5-nitrobenzaldehyd (GC: 99,3 Fl.% 2,5 Isomeres, 0,7 Fl .% 2,3 Isomeres).

### Beispiel 2

10 g 2-Chlor-5-nitrobenzaldehyds und 2-Chlor-3-nitrobenzaldehyd (GC: 91 Fl.% 2,5 Isomeres, 8,7 Fl.% 2,3 Isomeres) werden wie in Beispiel 1, jedoch mit einem Ethanol/Wasser-Gemisch im Volumenverhältnis von 1 : 1 behandelt. Man erhält 7,9 g (87 %) 2-Chlor-5-nitrobenzaldehyd (GC: 99,9 Fl .% 2,5 Isomeres, Spur 2,3 Isomeres).

### Beispiel 3

10 g 2-Chlor-5-nitrobenzaldehyds und 2-Chlor-3-nitrobenzaldehyd (GC: 91 Fl.% 2,5 Isomeres, 8,7 Fl.% 2,3 Isomeres) werden wie in Beispiel 1, jedoch mit einem Ethanol/Wasser-Gemisch im Volumenverhältnis von (1,5 : 1) behandelt. Man erhält 7,4 g (81 %) 2-Chlor-5-nitrobenzaldehyd (GC: 99,9 Fl.% 2,5 Isomeres, Spur 2,3 Isomeres).

### Beispiel 4

10 g 2-Chlor-5-nitrobenzaldehyds und 2-Chlor-3-nitrobenzaldehyd (GC: 91 Fl.% 2,5 Isomeres, 8,7 Fl.% 2,3 Isomeres) werden wie in Beispiel 1, jedoch in 50 ml Ethanol suspendiert und 50 ml Wasser zugetropft. Man rührt 25 min bei Raumtemperatur, kühlt auf 0°C ab und saugt den Feststoff ab. Man erhält 8,6 g (95 %) 2-Chlor-5-nitrobenzaldehyd (GC: 99,9 Fl.% 2,5 Isomeres, Spur 2,3 Isomeres).

### Beispiel 5

624 g eines Isomerengemisches aus 2-Chlor-5-nitrobenzaldehyds und 2-Chlor-3-nitrobenzaldehyd (GC: 91,7 Fl.% 2,5 Isomeres, 2,2 Fl.% 2,3 Isomeres, Rest andere Verunreinigungen) werden in 1 800 g Aceton bei 0°C vorgelegt und 1 200 ml Wasser innerhalb 30 min zugetropft. Man rührt 1 Stunde bei 0°C, saugt ab und wäscht mit kaltem Wasser nach. Man erhält 580 g (99 %) Isomerengemisch aus 2-Chlor-5-nitrobenzaldehyd und 2-Chlor-3-nitro-benzaldehyd (GC: 98,3 Fl.% 2,5 Isomeres, 0,3 Fl.% 2,3 Isomeres).

### Beispiel 6

9 g eines Isomerengemisches aus 2-Chlor-5-nitrobenzaldehyd und 2-Chlor-3-nitrobenzaldehyd (GC: 94,1 Fl.% 2,5 Isomeres, 5 Fl.% 2,3-Isomeres) werden in 50 g Methanol bei Raumtemperatur vorgelegt und in 10 min 30 g Petrolether zugetropft. Man rührt 45 min bei Raumtemperatur und 30 min bei 5 bis 10°C, dann wird abgesaugt und getrocknet. Man erhält 7 g (83 %) 2-Chlor-5-nitrobenzaldehyd (GC: 100 Fl.% 2, 5-Isomeres).

### Beispiel 7

2 kg eines Isomerengemisches aus 2-(2-Chlor-5-nitrophenyl)-1,3-dioxolan und 2-(2-Chlor-3-nitrophenyl)-1,3-dioxolan (HPLC (LiChrosorb RP 18, MeOH : H₂O : H₃PO₄ = 75 : 25 : 0,1 (V/V/V), 254 nm, 1ml/min) : 94,7 Fl.% 2,5 Isomeres, 2.1 Fl.% 2,3 Isomeres, Rest andere Verunreinigungen) werden mit Methanol/Wasser (Volumenverhältnis: 3 : 1) bei Raumtemperatur ausgewaschen, abgesaugt und bei 40 °C im Vakuum getrocknet. Man erhält 1810 g (95 %) 2-(2-Chlor-5-nitrophenyl)-1,3-dioxolan (HPLC: 99.1 % 2,5 Isomeres).

### Beispiel 8 (Vergleichsbeispiel nach DE-A-37 28 926)

174 g eines Isomerengemisches aus 2-Chlor-5-nitrobenzaldehyd und 2-Chlor-3-nitrobenzaldehyd (GC: 91,1 Fl.% 2,5 Isomeres, 8,7 Fl.% 2,3 Isomeres) werden in 445 g dest. Wasser vorgelegt, mit Natriumhydroxid neutralisiert, mit 10 g Benzyldimethyldodecylammoniumbromid versetzt und 30 min auf Rückfluß erhitzt. Die Emulsion wird 2,5 Stunden bei 40°C gerührt. Der bei Raumtemperatur ausgefallene 2-Chlor-5-nitrobenzaldehyd wird abgesaugt, mit Wasser gewaschen und im Vakuum getrocknet. Das Isomerengemisch wird quantitativ zurückerhalten. (GC: 91,9 Fl.% 2,5 Isomeres, 8,1 Fl.% 2,3 Isomeres).

## Patentansprüche

1. Verfahren zur Reinigung von 2-Chlor-5-nitrobenzaldehyd oder dessen Acetalen aus ihren 2,5- und 2,3-Isomerengemischen bei Temperaturen von (-10) bis +140°C, dadurch gekennzeichnet, daß man die Isomerengemische in einem C₁-C₂₀-Alkanol, einem Keton, einem Ester, einem Ether, einem cyclischen Ether, einem aromatischen Kohlenwasserstoff oder Mischungen dieser oder in Mischungen mit aliphatischen Kohlenwasserstoffen, Wasser oder Mischungen dieser suspendiert.

2. Verfahren nach Anspruch 1, dadurch gekennzeichnet, daß man im pH-Bereich von 4 bis 10 arbeitet.

3. Verfahren nach Anspruch 1, dadurch gekennzeichnet, daß man im pH-Bereich von 6 bis 8 arbeitet.

4. Verfahren nach Anspruch 1, dadurch gekennzeichnet, daß man 50 bis 99 Gew.-% des Isomerengemisches in der Suspension als Feststoff hält.

5. Verfahren nach Anspruch 1, dadurch gekennzeichnet, daß man 60 bis 98 Gew.-% des Isomerengemisches in der Suspension als Feststoff hält.

6. Verfahren nach Anspruch 1, dadurch gekennzeichnet, daß man 70 bis 95 Gew.-% des Isomerengemisches in der Suspension als Feststoff hält.

7. Verfahren nach Anspruch 1, dadurch gekennzeichnet, daß man bei Temperaturen von 0 bis 100°C suspendiert.

8. Verfahren nach Anspruch 1, dadurch gekennzeichnet, daß man bei Temperaturen von 10 bis 40°C suspendiert.

## Claims

1. A process for purifying 2-chloro-5-nitrobenzaldehyde or its acetals from their 2,5- and 2,3-isomer mixtures at from (-10) to +140°C, which comprises suspending the isomer mixtures in a C₁-C₂₀-alkanol, a ketone, an ester, an ether, a cyclic ether, an aromatic hydrocarbon or mixtures thereof or in mixtures with aliphatic hydrocarbons, water or mixtures thereof.

2. A process as claimed in claim 1, which is carried out in a pH range from 4 to 10.

3. A process as claimed in claim 1, which is carried out in a pH range from 6 to 8.

4. A process as claimed in claim 1, wherein 50-99 % by weight of the isomer mixture is kept in the suspension as solid.

5. A process as claimed in claim 1, wherein 60 - 98 % of the isomer mixture is kept in the suspension as solid.

6. A process as claimed in claim 1, wherein 70 - 95 % by weight of the isomer mixture is kept in the suspension as solid.

7. A process as claimed in claim 1, wherein suspension is carried out at from 0 to 100°C.

8. A process as claimed in claim 1, wherein suspension is carried out at from 10 to 40°C.

## Revendications

1. Procédé d'épuration du 2-chloro-5-nitrobenzaldéhyde ou de ses acétals à partir de leurs mélanges d'isomères 2,5 et 2,3, a des températures de (-10) à +140°C, caractérisé en ce que l'on met les mélanges d'isomères en suspension dans un alcanol en C₁ à C₂₀, une cétone, un ester, un éther, un éther cyclique, un hydrocarbure aromatique, ou des mélanges de ces composés, ou dans des mélanges avec des hydrocarbures aliphatiques, de l'eau, ou des mélanges de ceux-ci.

2. Procédé suivant la revendication 1, caractérisé en ce que l'on travaille dans une plage de pH de 4 à 10.

3. Procédé suivant la revendication 1, caractérisé en ce que l'on travaille dans une plage de pH de 6 à 8.

4. Procédé suivant la revendication 1, caractérisé en ce que l'on maintient de 50 à 99% en poids din mélange d'isomerès dans la suspension sous forme de substances solides.

5. Procédé suivant la revendication 1, caractérisé en ce que l'on maintient de 60 à 98% en poids du mélange d'isomères dans la suspension sous forme de substances solides.

6. Procédé suivant la revendication 1, caractérisé en ce que l'on maintient de 70 à 95% en poids du mélange d'isomères dans la suspension sous forme de substances solides.

7. Procédé suivant la revendication 1, caractérisé en ce que l'on opère la mise en suspension à des températures de 0 à 100°C.

8. Procédé suivant la revendication 1, caractérisé en ce que l'on opère la mise en suspension à des températures de 10 à 40°C.
